# EUROPEAN PATENT SPECIFICATION

(11) **EP 2 215 993 B1**
(45) Date of publication and mention of the grant of the patent: **12.03.2014**
(21) Application number: 10151171.5
(22) Date of filing: 20.01.2010
(51) Int. Cl.: A61F 5/00

(54) **ORTHOPAEDIC DEVICE FOR SUPPORTING AND GUIDING AN ARTICULATION**
Orthopädische Vorrichtung zur Unterstützung und Führung eines Gelenks
Appareil orthopédique pour supporter et guider une articulation

(30) Priority: 09.02.2009 IT MI20090165
(43) Date of publication of application: 11.08.2010
(73) Proprietor: Ro+Ten S.r.l., 20123 Milano (IT)
(72) Inventor: Bernareggi, Aldo, 20045, Besana in Brianza MB (IT); Ravese, Mauro, 20050, Correzzana MB (IT); Turconi, Francesco, 20052, Monza MB (IT)
(74) Representative: Branca, Emanuela

(56) References cited:
- EP-A- 0 629 362
- DE-A1- 3 835 927
- US-A- 3 935 858
- US-A- 5 865 776
- US-A1- 2007 130 665

## Description

The present invention refers to an orthopaedic device for supporting and guiding an articulation.

In particular, it refers to a knee brace, but also to other orthopaedic devices such as ankle supports, corsets, patella stabilizers and others, comprising a three-dimensional tubular element with non-constant section, possibly stiffened by longitudinal supports, suitable for being wrapped around a limb or a part of the torso.

For example, there are known knee braces produced in extensible textile material, at the sides of which there are containment pockets, or seats, sewn and inside of which there are enclosed stiffening rods or shafts with a joint arranged at the articulation, which can be limited or locked in its movement. Said devices are made up of a strap part, a stiffening part and belts, generally two, arranged one above and the other below the articulation, which are used to make the stiffening device more closely fixed to the limb for which it is intended. The belts are generally sewn at one of their ends to the strap part so that they keep their position, always being ready to be stretched, at the opposite end, once the knee brace is fitted.

A knee brace of this type is described for example in US-A-5.865.776.

The structure, as described above, in theory is adequate for the task that it has to perform, but in reality, given the irregular, not perfectly cylindrical, and often variable configuration of a leg at the knee, proves to be of limited effectiveness.

These problems are worsened by the variability of general dimensions, muscle mass, fatty tissue and whatever else of the user, which makes the real dimensions of the limb on which the knee brace has to be fitted unpredictable. As well as resulting in not very effective strapping with compromised mechanical characteristics, such solutions often have other drawbacks including uncomfortable points of pressure at one of the edges of the bands, in particular the edge arranged on the widest part of the conical frustum, the migration of the knee brace along the limb, abrasion and others.

It should also be observed that, in order to be effective, the bands must not be elastic, but in this way they cannot adapt to non-cylindrical shapes. However, if they are elastic, they create a "tourniquet" effect. It is therefore always a case of seeking a compromise.

Another example of an orthopaedic device is disclosed in the document US 2007/0130665.

The problems described above for exemplifying purposes for knee braces are encountered in an analogous way in other orthopaedic devices for supporting and guiding an articulation, such as ankle supports, corsets or other.

The purpose of the present invention is to make an orthopaedic device for supporting and guiding an articulation that avoids the drawbacks described earlier.

Another purpose of the present invention is to make an orthopaedic device for supporting and guiding an articulation comprising a structure able to mechanically adapt to every type of build both when static and dynamically, since it is adaptable in real time if required by muscle dilations and contractions, by the displacement of body fat, or other.

Another purpose of the present invention is to make an orthopaedic device for supporting and guiding an articulation that is particularly simple and functional, with low costs.

These purposes according to the present invention are accomplished by making an orthopaedic device for supporting and guiding an articulation as outlined in claim 1.

Further characteristics are foreseen in the dependent claims.

The characteristics and advantages of an orthopaedic device for supporting and guiding an articulation according to the present invention shall become clearer from the following description, given as an example and not for limiting purposes, referring to the attached schematic drawings, in which:
figures 1a, 1b and 1c are respectively front, rear and side views of an orthopaedic device for supporting and guiding an articulation, in the example a knee brace, according to the present invention;
figure 2a shows an enlarged detail of the double return buckle, which also closes the orthopaedic device according to the invention;
figures 2b and 2c are exploded views of the buckle of figure 2a seen from both sides;
figure 3 shows an enlarged detail of the containment pocket of the orthopaedic device according to the invention, which also makes a through opening for the bands.

With reference to the figures, an orthopaedic device for supporting and guiding an articulation is shown, wholly indicated with 10, which in the example illustrated as an example and not for limiting purposes is a knee brace.

The orthopaedic device for supporting and guiding an articulation 10 comprises a three-dimensional tubular element 12 with non-constant section and at least one band 13 for fastening the tubular element 12.

The three-dimensional tubular element 12 constitutes the frusto-conical elastic strapping part, suitable for being fitted onto the limb or onto the torso part to be strapped.

On the outer surface of the three-dimensional tubular element 12 one or more support and sliding guides 14 are also provided for each of the fastening bands 13, which can be one or more according to the longitudinal extension of the three-dimensional tubular element 12 and the tightness level required by the orthopaedic application.

The support and sliding guides 14 hold the fastening band 13 in position, whilst still allowing it to slide freely in the same guides 14 in the circumferential direction with respect to the tubular element 12, when the band 13 is pulled to fit the knee brace and during the subsequent tightening. The fact that the fastening band 13 is not fixed to the three-dimensional tubular element through sewing ensures uniformity of strapping and no tendency to make the knee brace rotate when the band 13 is tightened.

The orthopaedic device 10, according to the invention also comprises a double return buckle 15 for symmetrically tightening free opposite ends 16 of the fastening band 13.

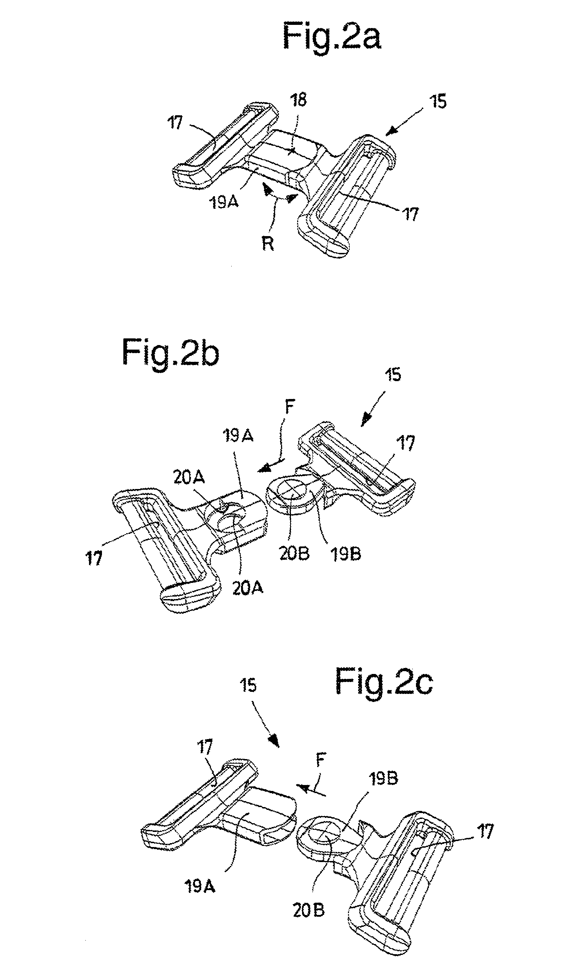

As shown in the detail of figure 2a, the double return buckle 15 comprises two slots 17 pivotally hinged together in an articulated manner at a joint 18 that allows the relative rotation of the two slots 17, indicated with the arrows R, to adapt with continuity to the conicity of the limb or of the torso portion to which the orthopaedic device 10 is applied.

The return buckle is made in two parts, advantageously to facilitate assembly and/or the first application by the doctor onto the patient's limb. For this purpose, as shown as an example in the exploded views of figures 2b and 2c, which show the double buckle 15 from both sides, the joint 18 is made through matching fastening means, suitable for being stably inserted one into the other, each forming a unit with a slot 17.

The slots 17, indeed, are integrally connected, respectively, to a containment end 19A and to an insertion end 19B, in which the containment end 19A consists of a pocket comprising a circular seat 20A on both sides and in which the insertion end 19B consists of a plane comprising a projection with circular plan 20B on both sides, matching the circular seat 20A, to form the joint 18.

According to the preferred embodiment shown, the circular projections 20B have increasing height in the coupling direction, as schematised in figures 2b and 2c with the arrows F, to facilitate insertion by pressure and at the same time prevent separation by traction.

The opposite ends 16 of the fastening band 13 can each be inserted in the corresponding slot 17 of the double buckle 15 and fixedly connected on itself in at the desired size through removable fastening means 25, like for example Velcro^{™}_{.}

The jointed double buckle 15 has the task of detecting, during the act of strapping, the conicity of the limb or of the body portion in the section in which the strapping is taking place, thus allowing the band to take up a position of planarity with respect to the limb or to the body, avoiding points of pressure and undesired rubbing. It is thus possible to obtain effective strapping with straps not subject to stretching, which is an undesired factor that could be necessary without the characteristics of adaptability of the orthopaedic device 10 according to the invention.

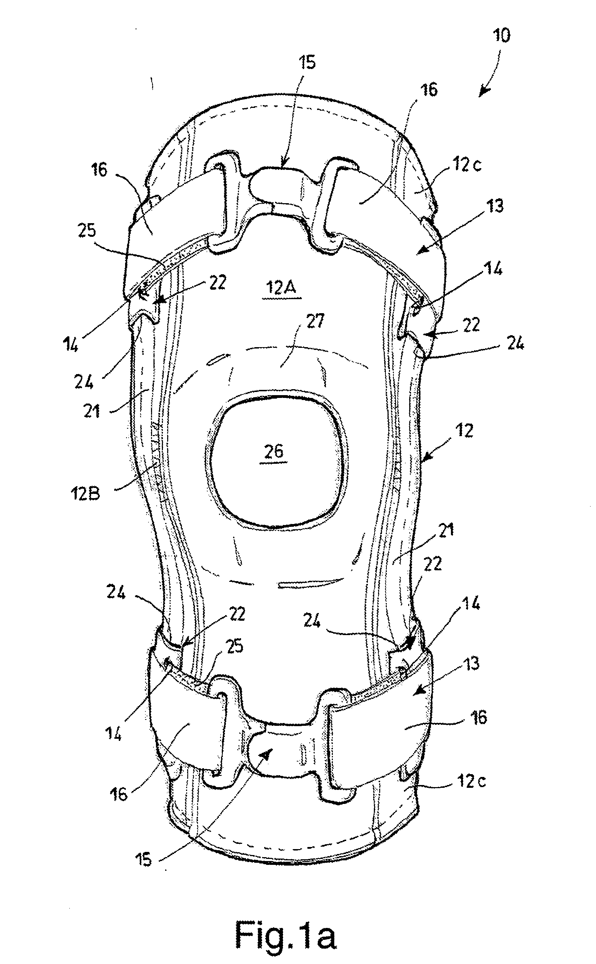

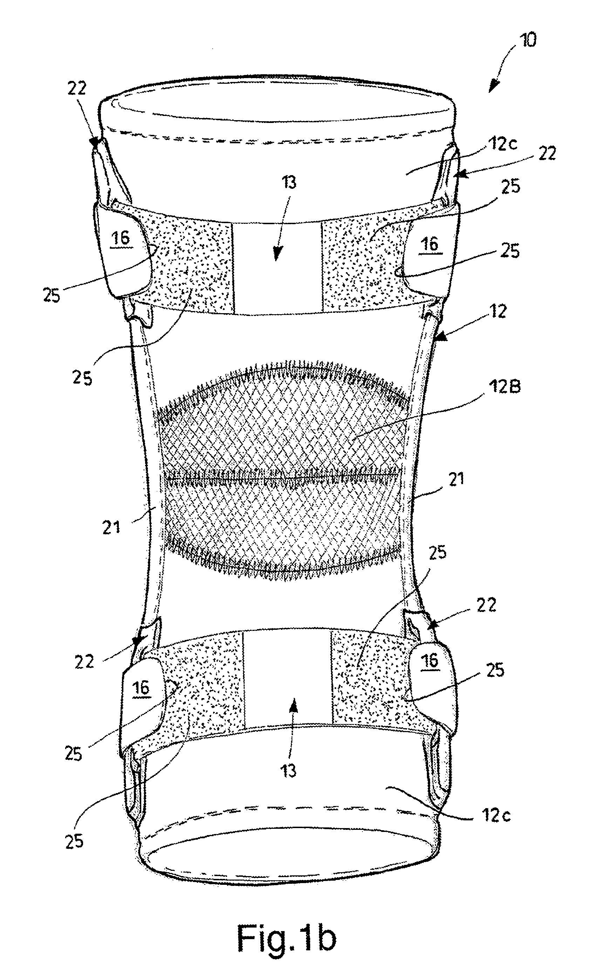

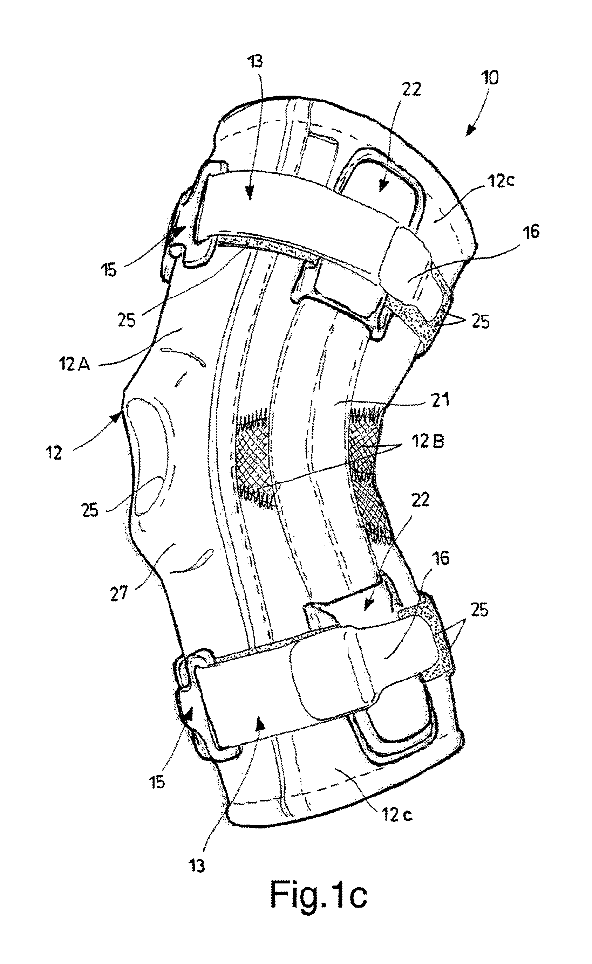

Moreover, the orthopaedic device 10 according to the invention can comprise longitudinal stiffening supports 21, such as articular rods, or rods for example in a spiral, shown in figure 1 sewn in the fabric. The opposite ends of the longitudinal stiffening supports 21 are inserted and held in position in containment pockets 22.

The containment pockets 22, preferably made of semi-rigid plastic or thermoplastic material, comprise, along at least part of the perimeter, a channel 23 to facilitate the sewing of the containment pocket 22 to the tubular element 12.

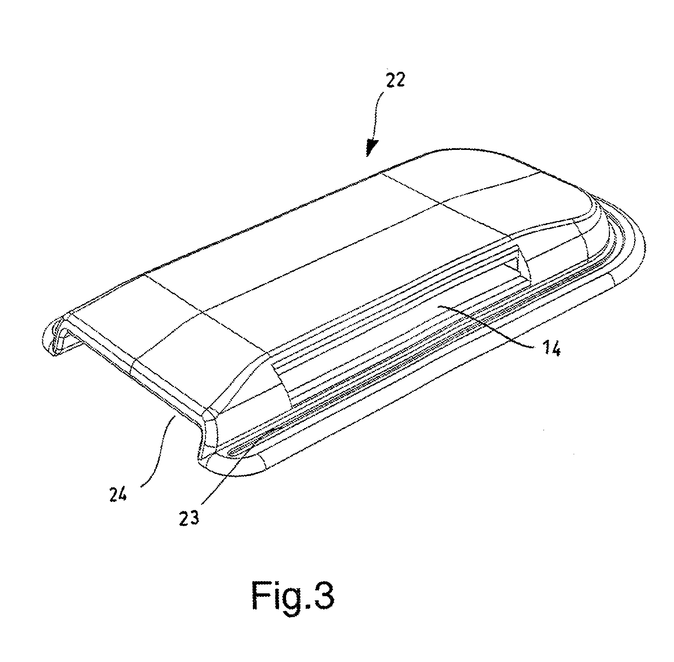

The channel 23 preferably extends over the entire perimeter if it has to contain articulated shafts or else it can extend just over three sides to contain the rods, as shown in figure 3.

According to the preferred embodiment of the containment pockets 22, shown in figure 1 and in the detail of figure 3, each containment pocket 22 also comprises a guide 14 for support and circumferential sliding of the fastening bands 13, arranged on the outer surface of a seat 24 for the end of the stiffening support 21.

According to another embodiment, not shown, the support and sliding guides 14 and the containment pockets 22 could be applied separately to the outer surface of the tubular element 12.

In particular, in the case of a knee brace, shown in figure 1, the tubular element 12 comprises four parts, preferably made from breathable material with characteristics variable with respect to each other depending on the mechanical requirements.

The knee brace comprises, according to the illustrated preferred embodiment, a front part 12A extending over the entire height of the tubular element 12. At the rear, on the other hand, it is divided in height into three areas, in which a central part 12B, subject to compression in the case of flexure, consists of one or more inserts made from more compressible and padded material to be more comfortable on the sensitive skin in the recess of the joint. Such characteristics of the rear central part also avoid a mass of material forming. At the rear, upper and lower parts 12C are made from the same material as the front part 12A.

In the other orthopaedic products according to the invention, the same principle of using a part of more compressible material that is padded in delicate areas subject to flexure is applied.

The parts are generally sewn together, but they could also be made with other constructive techniques.

Moreover, according to what is illustrated for the knee brace, the front part could be provided with a patella hole 26 surrounded by a patella stabiliser 27 according to what is known.

The orthopaedic device for supporting and guiding an articulation object of the present invention has the advantage of eliminating the tourniquet effect, instead providing areas of pressure that are well distributed with respect to the area of coverage of the fastening bands and of the stiffening supports.

Moreover, the orthopaedic device according to the invention allows the user to obtain the aforementioned benefits, irrespective of the size and configuration of the limb on which the device is arranged.

Moreover, advantageously, the orthopaedic device can easily be adjusted, since the elements in synergy will automatically take up the ideal arrangement for the limb or the body portion in question.

A further advantage consists of the low risk of undergoing twisting during the tightening and closing of the bands and the low possibility of undesired migration of the device into an incorrect position.

The orthopaedic device for supporting and guiding an articulation thus conceived can undergo numerous modifications and variants, all of which are covered by the invention; moreover, all of the details can be replaced with technically equivalent elements. In practice, the materials used, as well as the sizes, can be whatever according to the technical requirements.

## Claims

1. Orthopaedic device for supporting and guiding an articulation, comprising a three-dimensional tubular element (12) with non-constant section and at least one band (13) for fastening the tubular element (12), said at least one fastening band (13) being circumferentially mobile with respect to the tubular element (12) and having two free opposite ends (16), the orthopaedic device comprising, on the tubular element (12), guides (14) for supporting and circumferential sliding of said at least one fastening band (13) with respect to the tubular element (12) and a double return buckle (15) for symmetrically tightening of said free opposite ends (16) of said at least one fastening band (13), wherein said free opposite ends (16) are each insertable into a slot (17) of said double return buckle (15) and able to be fixedly connected to itself through removable fastening means (25), the orthopaedic device comprising longitudinal stiffening supports (21) and containment pockets (22) for opposite ends of said longitudinal stiffening supports (21), wherein said containment pockets (22) are applied outside the tubular element (12),
**characterised in that** each of said containment pockets (22) also comprises said guide (14) for support and circumferential sliding of said at least one fastening band (13), arranged on the outer surface of a seat (24) for the end of said stiffening support (21).

2. Orthopaedic device according to claim 1, **characterised in that** said double return buckle (15) comprises a joint (18) for pivoting said slots (17) in an articulated manner to adapt with continuity to the conicity of a limb or of a body portion on which the tubular element (12) is applied.

3. Orthopaedic device according to claim 2, **characterised in that** said slots (17) are each integrally connected, respectively, to a containment end (19A) and to an insertion end (19B), wherein said containment end (19A) comprises a circular seat (20A) on both sides and wherein said insertion end (19B) comprises, on both sides, a projection with circular plan (20B), matching said circular seat (20A).

4. Device according to claim 3, **characterised in that** said circular projections (20B) have an increasing height in the coupling direction to facilitate pressure-insertion and prevent the traction-separation.

5. Orthopaedic device according to claim 1, **characterised in that** said containment pockets (22) are made of semi-rigid plastic material and comprise, along at least part of the perimeter, a channel (23) for sewing to the tubular element (12).

6. Orthopaedic device according to claim 1, **characterised in that** said tubular element (12) comprises many parts (12A, 12B, 12C) with characteristics variable with respect to each other depending on the mechanical requirements.

7. Device according to claim 6, **characterised in that** said tubular element (12) comprises a front part (12A) extending along the entire height of said tubular element (12) and at the rear, being divided in height into three areas, a central part (12B), subjected to compression in the case of flexure and consisting of at least one insert of more compressible and padded material, as well as upper and lower parts (12C) of the same material as said front part (12A).

8. Orthopaedic device according to any one of the previous claims, **characterised in that** it is a knee brace.

## Patentansprüche

1. Orthopädische Vorrichtung zur Unterstützung und Führung eines Gelenks, die ein dreidimensional röhrenförmiges Element (12) mit einem nicht konstanten Querschnitt und mindestens ein Band (13) zum Befestigen des röhrenförmigen Elements (12) umfasst, wobei dieses mindestens eine Befestigungsband (13) zirkumferenziell gegenüber dem röhrenförmigen Element (12) beweglich ist und zwei freie entgegengesetzte Enden (16) hat, wobei die orthopädische Vorrichtung auf dem röhrenförmigen Element (12) Führungen (14) zum Tragen und zirkumferenziellen Gleiten des mindestens einen Befestigungsbands (13) gegenüber dem röhrenförmigen Element (12) und eine Doppelrückführschnalle (15) zum symmetrischen Straffen der freien entgegengesetzten Enden (16) des mindestens einen Befestigungsbands (13) umfasst, wobei diese freien entgegengesetzten Enden (16) jedes in einen Schlitz (17) dieser Doppelrückführschnalle (15) eingeführt und durch lösbare Befestigungsmittel (25) fest miteinander verbunden werden können, wobei die orthopädische Vorrichtung längslaufende Versteifungsstützen (21) und Rückhaltetaschen (22) für entgegengesetzte Enden dieser längslaufenden Versteifungsstützen (21) umfasst, wobei diese Rückhaltetaschen (22) außen an dem röhrenförmigen Element (12) angebracht sind, **dadurch gekennzeichnet, dass** jede dieser Rückhaltetaschen (22) außerdem die Führung (14) zum Tragen und zirkumferenziellen Gleiten des mindestens einen Befestigungsbands (13) umfasst, die auf der Außenfläche eines Sitzes (24) für das Ende der Versteifungsstütze (21) angeordnet ist.

2. Orthopädische Vorrichtung nach Anspruch 1, **dadurch gekennzeichnet, dass** die Doppelrückführschnalle (15) ein Gelenk (18) zum gelenkigen Drehen der Schlitze (17) umfasst, damit sie sich kontinuierlich an die konische Form einer Gliedmaße oder eines Körperteils anpassen, an der bzw. dem das röhrenförmige Element (12) angebracht ist.

3. Orthopädische Vorrichtung nach Anspruch 2, **dadurch gekennzeichnet, dass** die Schlitze (17) jeweils integral mit einem Aufnahmeendstück (19A) und einem Einführendstück (19B) verbunden sind, wobei das Aufnahmeendstück (19A) eine kreisförmige Aufnahme (20A) auf beiden Seiten umfasst und wobei das Einführendstück (19B) auf beiden Seiten einen Vorsprung mit einer kreisförmigen Fläche (20B) umfasst, die der kreisförmigen Aufnahme (20A) entspricht.

4. Vorrichtung nach Anspruch 3, **dadurch gekennzeichnet, dass** die kreisförmigen Vorsprünge (20B) eine in der Kopplungsrichtung zunehmende Höhe aufweisen, um das Einführen mit Druck zu erleichtern und das Lösen durch Zug zu verhindern.

5. Orthopädische Vorrichtung nach Anspruch 1, **dadurch gekennzeichnet, dass** die Rückhaltetaschen (22) aus einem halbstarren Kunststoff bestehen und entlang zumindest eines Teils des Umfangs einen Kanal (23) zum Nähen an das röhrenförmige Element (12) umfassen.

6. Orthopädische Vorrichtung nach Anspruch 1, **dadurch gekennzeichnet, dass** das röhrenförmige Element (12) viele Teile (12A, 12B, 12C) mit voneinander in Abhängigkeit von den mechanischen Anforderungen verschiedenen Eigenschaften umfasst.

7. Vorrichtung nach Anspruch 6, **dadurch gekennzeichnet, dass** das röhrenförmige Element (12) einen vorderen Teil (12A) umfasst, der sich entlang der ganzen Höhe des röhrenförmigen Elements (12) erstreckt, und auf der Rückseite in der Höhe in drei Bereiche unterteilt ist, einen mittleren Teil (12B), der im Falle einer Beugung Kompression ausgesetzt ist und aus mindestens einem Einsatz aus einem komprimierbareren und gepolsterten Material besteht, sowie obere und untere Teile (12C) aus dem gleichen Material wie der vordere Teil (12A).

8. Orthopädische Vorrichtung nach einem der vorhergehenden Ansprüche **dadurch gekennzeichnet, dass** sie eine Kniestütze ist.

## Revendications

1. Dispositif orthopédique pour supporter et guider une articulation, comprenant un élément tubulaire tridimensionnel (12) avec une section non constante et au moins une bande (13) pour fixer l'élément tubulaire (12), ladite au moins une bande de fixation (13) étant mobile de manière circonférentielle par rapport à l'élément tubulaire (12) et ayant deux extrémités libres opposées (16), le dispositif orthopédique comprenant, sur l'élément tubulaire (12), des guides (14) pour le support et le coulissement circonférentiel de ladite au moins une bande de fixation (13) par rapport à l'élément tubulaire (12) et une boucle à double retour (15) pour serrer symétriquement lesdites extrémités libres opposées (16) de ladite au moins une bande de fixation (13), dans lequel lesdites extrémités libres opposées (16) peuvent être insérées chacune dans une fente (17) de ladite boucle à double retour (15) et sont adaptées pour être connectées fixement à elle-mêmes par le biais de moyens de fixation détachables (25), le dispositif orthopédique comprenant des supports de renforcement longitudinaux (21) et des poches de confinement (22) pour des extrémités opposées desdits supports de renforcement longitudinaux (21), dans lequel lesdites poches de confinement (22) sont appliquées à l'extérieur de l'élément tubulaire (12), **caractérisé en ce que** chacune desdites poches de confinement (22) comprend également ledit guide (14) pour le support et le coulissement circonférentiel de ladite au moins une bande de fixation (13), disposé sur la surface extérieure d'un siège (24) pour l'extrémité dudit support de renforcement (21).

2. Dispositif orthopédique selon la revendication 1, **caractérisé en ce que** ladite boucle à double retour (15) comprend un joint (18) pour le pivotement desdites fentes (17) d'une manière articulée pour s'adapter avec continuité à la conicité d'un membre ou d'une partie de corps sur lequel l'élément tubulaire (12) est appliqué.

3. Dispositif orthopédique selon la revendication 2, **caractérisé en ce que** lesdites fentes (17) sont connectées chacune d'une seule pièce, respectivement, à une extrémité de confinement (19A) et à une extrémité d'insertion (19B), dans lequel ladite extrémité de confinement (19A) comprend un siège circulaire (20A) sur les deux côtés et dans lequel ladite une extrémité d'insertion (19B) comprend, des deux côtés, une saillie avec plan circulaire (20B), coïncidant avec ledit siège circulaire (20A).

4. Dispositif orthopédique selon la revendication 3, **caractérisé en ce que** lesdites saillies circulaires (20B) ont une hauteur croissante dans la direction d'accouplement pour faciliter l'insertion par pression et empêcher la séparation par traction.

5. Dispositif orthopédique selon la revendication 1, **caractérisé en ce que** lesdites poches de confinement (22) sont réalisées en matériau plastique semi-rigide et comprennent, le long d'au moins une partie du périmètre, un canal (23) pour la couture sur l'élément tubulaire (12).

6. Dispositif orthopédique selon la revendication 1, **caractérisé en ce que** ledit élément tubulaire (12) comprend de nombreuses parties (12A, 12B, 12C) avec des caractéristiques variables les unes par rapport aux autres en fonction des exigences mécaniques.

7. Dispositif orthopédique selon la revendication 6, **caractérisé en ce que** ledit élément tubulaire (12) comprend une partie frontale (12A) s'étendant sur toute la hauteur dudit élément tubulaire (12) et sur l'arrière, qui est divisé en hauteur en trois zones, une partie centrale (12B), soumise à une compression dans le cas d'une flexion et consistant en au moins une pièce rapportée en matériau rembourré et plus compressible, ainsi que des parties supérieure et inférieure (12C) du même matériau que ladite partie frontale (12A).

8. Dispositif orthopédique selon l'une quelconque des revendications précédentes, **caractérisé en ce qu'**il est une attelle de genou.
